# EUROPEAN PATENT SPECIFICATION

(11) **EP 2 289 424 B1**
(45) Date of publication and mention of the grant of the patent: **25.07.2012**
(21) Application number: 10184677.2
(22) Date of filing: 20.10.2001
(51) Int. Cl.: A61B 17/00, A61B 17/02, A61B 17/34

(54) **Wound retraction apparatus**
Wundretraktionsvorrichtung
Appareil pour rétraction des plaies

(43) Date of publication of application: 02.03.2011
(62) Divisional of application: 01985170.8
(73) Proprietor: APPLIED MEDICAL RESOURCES CORPORATION, Rancho Santa Margarita, CA 92688 (US)
(72) Inventor: Ewers, Richard C, Fullerton, CA 92833 (US); Brustad, John R, Dana Point, CA 92629 (US); Pingleton, Edward D, San Juan Capistrano, CA 92675 (US); Hilal, Nabil, Laguna Niguel, CA 92677 (US); Adlparvar, Payam, Lake Forest, CA 92630 (US); Taylor, Scott, Mission Viejo, CA 92692 (US); Dulak, Gary R, Newport Beach, CA 92663 (US); Dunn, Michael J, Santa Ana, CA 92706 (US); Morales, Norman, San Jose, CA 95126 (US); Hart, Charles C, Summerville, SC 29483-8949 (US); Bowes, Robert R. II, Trabuco Canyon, CA 92679 (US)
(74) Representative: Fitchett, Stuart Paul

(56) References cited:
- EP-A- 0 849 517
- EP-A1- 1 407 715
- WO-A-00/32116
- WO-A1-98/48724
- US-A- 5 514 133
- US-A- 6 090 043

## Description

### Background of the Invention

### Field of Invention

This invention relates generally to wound retraction and more specifically to wound retraction in a laparoscopic surgical procedure.

### Discussion of the Related Art

During laparoscopic surgery, it is desirable to inflate the abdominal cavity in order to increase the volume of the working space. This is accomplished with an insufflation gas which must be maintained at a pressure sufficient to inflate the abdomen. Maintaining the pressure of the insufflation gas is difficult when it is also desirable to insert instrumentation through the abdominal wall. If the surgeon is interested in inserting his or her hand in a hand-assisted laparoscopic procedure, the maintenance of insufflation pressure is even more difficult. Currently, several devices exist that accomplish this surgical need although they suffer from drawbacks such as difficult placement and cumbersome use. Thus, it is desirable that the wound be retracted, protected, and fixed while maintaining an insufflation seal. United States patent US 5,514,133 discloses an access device for endoscopic surgery, and PCT application number WO 00/32116 discloses a surgical device for both retracting and protecting a wound.

### Summary of the Invention

According to the present invention there is provided a surgical wound retractor adapted to dilate a wound, the retractor comprising: a first ring having a diameter greater than the desired diameter of the wound and being adapted for disposition interiorly of the wound; a second ring having a diameter greater than the desired diameter of the wound and being adapted for disposition exteriorly of the wound; a sheath disposed between the first ring and the second ring; and a plurality of retention elements disposed between the first ring and the second ring, the retention elements being adapted for disposition through the wound to exert a radial retraction force on the wound, the retention elements each comprise a distal end, a proximal end, and a plurality of engagement sites disposed between the proximal end and the distal end, wherein each engagement site is associated with a different retraction force, the distal ends of the retention elements are coupled to the first ring, and the second ring comprises a plurality of retainers, wherein each retainer is dimensioned and configured for engaging a selected one of the engagement sites to provide an associated radial retraction force and wherein each of the plurality of retention element engagement sites comprises a ladder rung and the plurality of retainers comprise hooks on which a selected ladder rung of a retainer may be hooked.

A wound retractor in accordance with the present invention allows the surgeon to easily locate a retractor and to provide a solid base for an instrument or hand seal. This retractor removes the tissue pressure from the wrist during hand-assisted laparoscopic surgery. It can also protect the tissue at the wound site, for example, from abrasion, bacteria or other contaminants organs, such as donor kidneys to be removed with minimal risk or damage. The retractor also opens the wound providing greater access to the operative site for instruments, such as the hand of the surgeon.

### Description of the Drawings

FIGS. 1 and 2 are perspective views illustrating the principle of operating a wound retractor;
FIG. 3 is a perspective view of a wound retractor in accordance with the present invention including multiple ladders with rungs providing a sheath with desired tension.

### Description of Preferred Embodiments

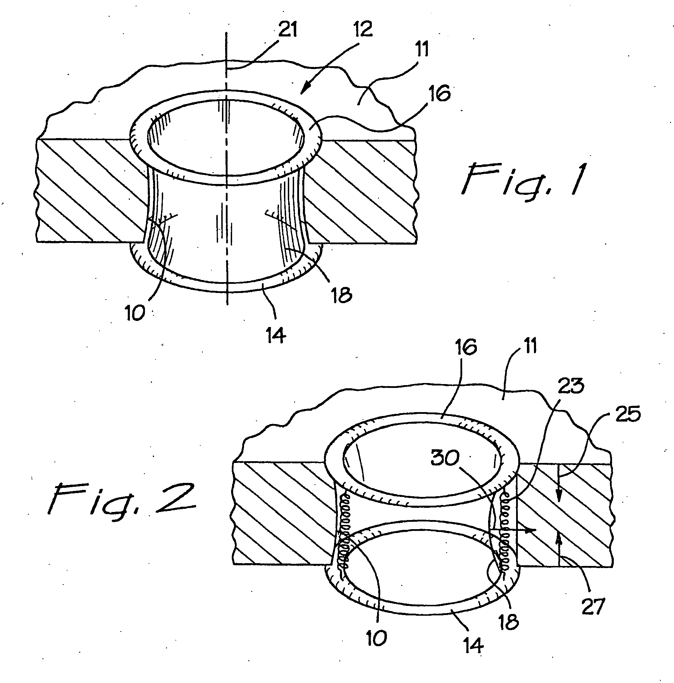

The basic concept of retracting and protecting a wound site is illustrated in the prospective view of Figure 1 wherein a wound 10 is formed in an abdominal wall 1 l. In this embodiment, a retractor 12 uses two rings 14 and 16 which are fixed to an elastic sheath 18. The sheath 18 has a generally cylindrical configuration and is disposed along an axis 21. The rings 14 and 16 are disposed in respective planes which extend radially of the axis 21.

The sheath 18 has elastomeric properties, but in its natural, unstretched state the two rings 14 and 16 are separate by a natural distance. The lower ring 14 is placed interiorly of the abdominal wall 11 and the upper ring 14 is stretched beyond the natural distance away from the lower ring. Once the elastic sheath 18 has been stretched to a distance greater than the abdominal wall thickness, the upper ring 16 is placed on the surface of the skin.

Since the diameters of the rings 14, 16 are greater than that desired for the wound site 10, they will have sufficient footing to maintain this tension between the two rings 14, 16. This tension is created by the elastic material that has been stretched and retained at a distance greater than the natural distance. It will be appreciated that in many embodiments, the sheath 18 can be formed of a non-elastic sheathing material. In a similar manner, the rings 14 and 16 may be provided with a rigid configuration or alternatively may be formed of an elastomeric material.

Figure 2 shows a simple schematic of the ring dynamics illustrated in Figure 1, with the retractor 12 operatively disposed across the abdominal wall 11. The elastic sheet sheath 18 acts as a circumferential spring 23 around the wound site 10 that evenly distributes the tension between the two rings 14 and 16, as represented by arrows 25 & 27. In addition, the elastic sheeting provides a radial retraction force 30 around the wound to enlarge the wound site 10 in order to facilitate the passage of instruments, such as the hand of the surgeon.

The amount of tension force between the two rings 14 and 16 can be controlled by the elastomeric proportion of the elastic sheath 18. In order to accommodate a larger range of abdominal wall thicknesses, a material with a higher elasticity can be chosen to allow for greater stretch.

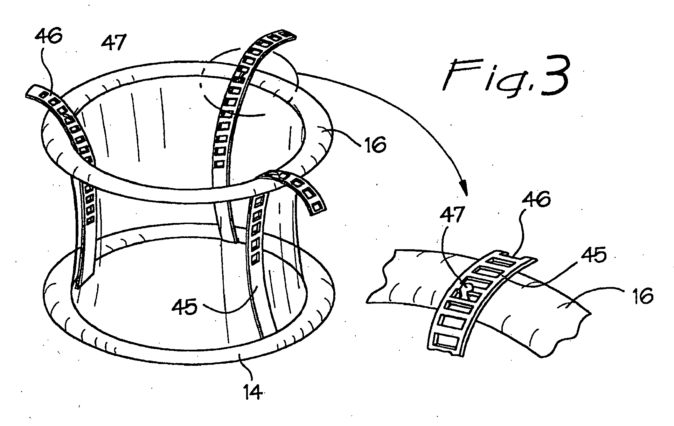

Figure 3 illustrates a wound retractor in accordance with the present invention having retention elements 45 in the form of ladders 45 with rungs 46, where the user simply hooks an appropriate rung 46 of a ladder 45 onto an associated hook 47 located on the outer ring 16.

A sealing cap 105 can be disposed over the outer ring 16d to facilitate laparoscopic surgery.

Those skilled in the art will envision other modifications within the scope of the present invention as defined by the following claims.

## Claims

1. A surgical wound retractor adapted to dilate a wound, the retractor comprising:
a first ring (14) having a diameter greater than the desired diameter of the wound and being adapted for disposition interiorly of the wound;
a second ring (16) having a diameter greater than the desired diameter of the wound and being adapted for disposition exteriorly of the wound;
a sheath (18) disposed between the first ring and the second ring; and
a plurality of retention elements (45) disposed between the first ring and the second ring, the retention elements being adapted for disposition through the wound to exert a radial retraction force on the wound, the retention elements each comprise a distal end, a proximal end, and a plurality of engagement sites disposed between the proximal end and the distal end, wherein each engagement site is associated with a different retraction force, the distal ends of the retention elements are coupled to the first ring, and the second ring comprises a plurality of retainers, wherein each retainer is dimensioned and configured for engaging a selected one of the engagement sites to provide an associated radial retraction force,
**characterized in that** each of the plurality of retention element engagement sites comprises a ladder rung (46) and the plurality of retainers comprise hooks on which a selected ladder rung (46) of a retainer (45) may be hooked.

## Patentansprüche

1. Ein chirurgischer Wundretraktor, der zur Dilatation einer Wunde angepasst ist, wobei der Retraktor umfasst:
einen ersten Ring (14) mit einem Durchmesser, der größer ist als der gewünschte Durchmesser der Wunde und der zur Anordnung innerhalb der Wunde angepasst ist;
einen zweiten Ring (16) mit einem Durchmesser, der größer ist als der gewünschte Durchmesser der Wunde und der zur Anordnung außerhalb der Wunde angepasst ist;
eine Schleuse (18), die zwischen dem ersten Ring und dem zweiten Ring angeordnet ist; und
eine Vielzahl von Halteelementen (45), die zwischen dem ersten Ring und dem zweiten Ring angeordnet sind, wobei die Halteelemente zur Anordnung durch die Wunde angepasst sind, um eine radiale Rückhaltekraft auf die Wunde auszuüben, wobei die Halteelemente jeweils ein distales Ende, ein proximales Ende und eine Vielzahl von Eingriffsstellen umfassen, die zwischen dem proximalen Ende und dem distalen Ende angeordnet sind, wobei jede Eingriffsstelle mit einer unterschiedlichen Rückhaltekraft verbunden ist, die distalen Enden der Halteelemente an den ersten Ring gekoppelt sind, und der zweite Ring eine Vielzahl von Halterungen umfasst, wobei jede Halterung zum Eingriff mit einer ausgewählten der Eingriffsstellen dimensioniert und konfiguriert ist, um eine damit verbundene radiale Rückhaltekraft bereitzustellen,
**gekennzeichnet dadurch, dass** jede der Vielzahl der Halteelement-Eingriffsstellen eine Leitersprosse (46) umfasst und dass die Vielzahl der Halterungen Haken umfassen, auf denen eine ausgewählte Leitersprosse (46) einer Halterung (45) angehakt sein kann.

## Revendications

1. Appareil pour rétraction des plaies adapté pour dilater une plaie, ledit appareil pour rétraction comprenant :
un premier anneau (14) ayant un diamètre supérieur au diamètre désiré de la plaie et étant adapté de manière à être disposé intérieurement à la plaie ;
un second anneau (16) ayant un diamètre supérieur au diamètre désiré de la plaie et étant adapté de manière à être disposé extérieurement à la plaie ;
une gaine (18) positionnée entre le premier anneau et le second anneau ; et
une pluralité d'éléments de retenue (45) positionnés entre le premier anneau et le second anneau, les éléments de retenue étant adaptés pour être disposés dans la plaie en vue d'exercer une force de rétraction radiale sur la plaie, les éléments de retenue comprenant chacun une extrémité distale, une extrémité proximale, et une pluralité de sites d'engagement disposés entre l'extrémité proximale et l'extrémité distale, dans lesquels chaque site d'engagement est associé à une force de rétraction différente, les extrémités distales des éléments de retenue sont couplés au premier anneau, et le second anneau comprend une pluralité de retenues, dans lequel chaque retenue est dimensionnée et configurée de manière à engager un des sites d'engagement sélectionné pour fournir une force de rétraction radiale associée,
**caractérisé en ce que** chacun de la pluralité des sites d'engagement des éléments de retenue comprend un barreau (46) et la pluralité de retenues comprend des crochets sur lesquels un barreau sélectionné (46) de la retenue (45) peut être accroché.
